# EUROPEAN PATENT APPLICATION

(11) **EP 1 616 582 A1**
(43) Date of publication of application: **18.01.2006**
(21) Application number: 04723494.3
(22) Date of filing: 26.03.2004
(51) Int. Cl.: A61L 15/18, A61K 33/00, A61P 43/00

(54) **NEW DRESSING MATERIAL PROMOTING RECOVERY OF SKIN WOUND**

(30) Priority: 09.04.2003 CN 03109623
(71) Applicant: Jiangsu Yenssen Biotech Co. Ltd., Jiangyin, Jiangsu 214431 (CN)
(72) Inventor: ZHOU, Laisheng, Jiangyin, Jiangsu 214431 (CN)
(74) Representative: de Saint-Palais, Arnaud Marie
(86) International application number: PCT/CN2004/000265
(87) International publication number: WO 2004/089430

(57) **Abstract**

The present invention discloses a novel dressing material for promoting the proliferation of epithelial cells and the use of the same in the treatment of wound surface of skin. The dressing material uses silicon and/or calcium inorganic elements as biologically active substances being capable of initiatively inducing the proliferation of epithelial cells of human skin, inhibiting inflammatory exudates from the wound surface, and promoting the rapid healing of wound surface of skin, and is in the dosage form of powder, ointment or patch. The dressing material can be used safely, economically and effectively in the treatment of large chronic wound surface such as incised wounds, contusions, burns, scalds, chemical burns, bedsores, various ulcers on the surface of skin, and the like.

## Description

### FIELD OF THE INVENTION

The present invention relates to a novel dressing material for promoting the proliferation of epithelial cells, to use of inorganic element(s) silicon and/or calcium in the preparation of such a dressing material, and to a method of promoting the proliferation of epithelial cells in vitro.

### BACKGROUND ART OF THE INVENTION

Various wounds of skin on the human body surface are common conditions inevitably occurring in the daily life, and usually include incised wounds, contusions, burns, chemical burns, and the like. The more delay of wound healing, the more difficult will scars disappear after wounds healing. As to large and chronic wound surface of skin such as bedsores and various skin ulcers of patients suffering diabetes, the rapid repair and growth of epithelia are particularly needed. Hence, the optimal treatment of various skin wounds is to control secondary bacterial infection and to promote rapid wound healing.

In the prior art, various antibacterial ointments, creams or powders are widely used as dressings for external use on human body surface. However, single or composite antibiotic dressings can merely control secondary bacterial infection, and will usually not exhibit biological activity of actively inducing the rapid growth of epithelial cells at the wound surface to result in the rapid healing of the wound surface. US Patent 6,262,020 discloses a topical wound therapeutic formulation containing hyaluronic acid for controlling the inflammatory reaction at the wound surface of skin. US Patent 5,190,917 and US Patent 5,290,762 discloses the anti-inflammatory effects of serine protease inhibitors, and said inhibitors are incorporated into a topical wound therapeutic formulation for inhibiting the inflammatory reaction at wound surface in US Patent 6,262,020. However, all of the topical formulations disclosed in these patents do not possess the function of actively inducing and promoting the rapid growth of epithelia at the wound surface. US Patent 5,604,200 discloses a topical wound dressing comprising plasma fibronectin. However, although this fibronectin could theoretically promote the adhesion of human cells, in the practical application, if the fibronectin is extracted from human plasma, the source will be limited and the plasma fibronectin will be too expensive to be used clinically, and if the fibronectin is obtained via genetic engineering process in vitro, it will not process a biological activity similar to that of the natural proteins in human body. In addition, both the natural and synthesized proteins can hardly be sterilized and may readily cause hypersensitivity in human body, and the stability of the protein can hardly be maintained under various situations, such as during production, storage, transportation, distribution and clinics. Hence, the idea of using biologically active proteins as active ingredients in a common topical formulation is lack of practical utility clinically.

Thus, dressing materials having sufficient biological stability and being capable of inducing the proliferation of epithelial cells and the wound healing are dramatically desired in the state of art.

### BRIEF SUMMARY OF THE INVENTION

The present invention is based on the inventor's long-term and in-depth researches for developing a topical biological material having activities of initiatively inducing the proliferation of epithelial cells, eliminating inflammation, and actively promoting the rapid healing of wound on human skin, and directly based on the inventor's pioneer discovery.

In the conventional concepts, all inorganic element materials are "inert", i.e., inorganic element materials do not possess the inducing activity of stimulating the growth of biological cells.

The inventor's pioneer discovery indicates that some inorganic element materials can also be used as chemical signals to selectively stimulate and regulate the growth and differentiation of cells.

Concretely, the inventor discovers that microparticles of the inorganic elements silicon and/or calcium have the biological activity of initiatively inducing the proliferation, differentiation and migration of human epithelial cells, can control pH at wound surface by neutralization to prevent inflammation, and have better performance in inducing the healing of epithelial wound and in preventing the secondary bacterial infection.

On the one hand, the present invention provides a dressing material for treating or alleviating diseases or conditions in need of promoting the proliferation and differentiation of epithelial cells, wherein said dressing material comprises an effective amount of microparticles of inorganic element silicon and/or calcium and optionally pharmaceutically acceptable carriers and/or excipients. Such a dressing material can actively induce the proliferation of epithelial cells and promote the rapid healing of wound.

On the other hand, the present invention further relates to the use of inorganic element silicon and/or calcium in the preparation of a dressing material for treating or alleviating diseases or conditions in need of promoting the proliferation or differentiation of epithelial cells.

Finally, the present invention provides a method of promoting the proliferation of epithelial cells in vitro, comprising applying an effective amount of silicon and/or calcium microparticles to said cells.

### BRIEF DESCRIPTION OF THE DRAWINGS

Fig. 1 is a scanning electron micrograph of a dressing material comprising silicon/calcium microparticles.
Fig. 2 is a photograph of cultured normal human epithelial cells under the induction of silicon/calcium microparticles.
Fig. 3 indicates the effects of silicon/calcium microparticles or combination thereof at different concentrations on promoting the proliferation of normal human epithelial cells, wherein Fig. 3a shows the proliferation folds of epithelial cells exposed to silicon and calcium elements for 12 days, and Fig. 3b shows the proliferation folds of epithelial cells exposed to silicon and calcium elements for 20 days.
Fig. 4 indicates the effects of silicon/calcium microparticles or combination thereof at different concentrations on inducing the synthesis and secretion of type-IV collagen in normal human epithelial cells, wherein Fig. 4a shows the level of type-IV collagen in epithelial cells exposed to silicon and calcium elements for 12 days, and Fig. 4b shows the level of type-IV collagen in epithelia exposed to silicon and calcium elements for 20 days.
Fig. 5 indicates the effects of silicon/calcium microparticles or combination thereof at different concentrations on inducing the synthesis and secretion of epithelial growth factor in normal human epithelial cells, wherein Fig. 5a shows the level of epithelial growth factor in epithelial cells exposed to silicon and calcium elements for 12 days, and Fig. 5b shows the level of epithelial growth factor in epithelial cells exposed to silicon and calcium elements for 20 days.
Fig. 6 is a photograph of the course of treatment using silicon/calcium microparticles dressing materials in a clinical case.

### DETAILED DESCRIPTION OF THE PREFERRED EMBODIMENTS

In one embodiment, the present invention provides a dressing material for treating or alleviating diseases or conditions in need of promoting the proliferation or differentiation of epithelial cells, wherein said dressing material comprises an effective amount of inorganic element silicon and/or calcium microparticles and optionally pharmaceutically acceptable carriers and/or excipients. In the dressing material of the present invention, the contents of inorganic element silicon and/or calcium microparticles can be widely changed or adjusted, such as from 0 to 100%, preferably from 0.5 to 90% by their or its weight within the mixture.

In the dressing material of the present invention, said inorganic element silicon could be in any inorganic substance form, including for example SiO₂, NaAlSiO₂, KAlSiO₂ or any combination thereof; and said inorganic element calcium could be in any inorganic substance form, including for example CaO, CaSO₄, CaPO₄, CaCl₂ or in any combination thereof.

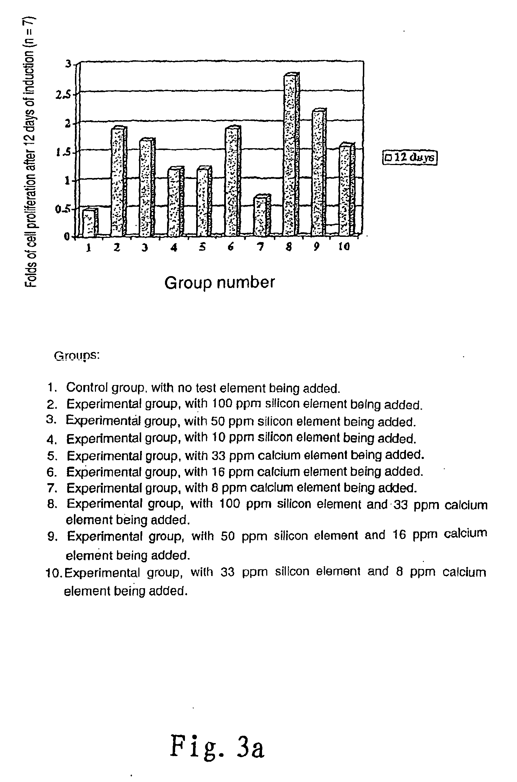

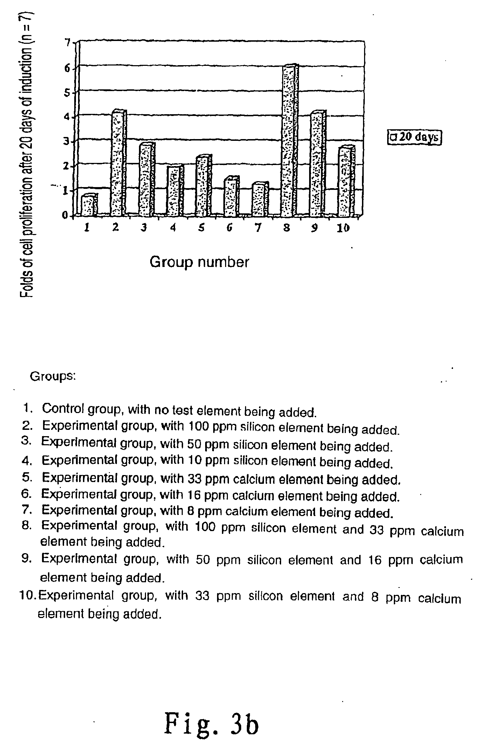

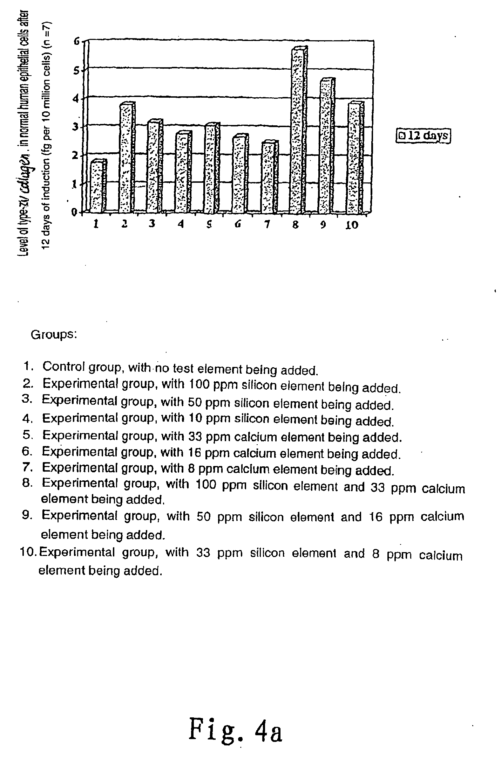

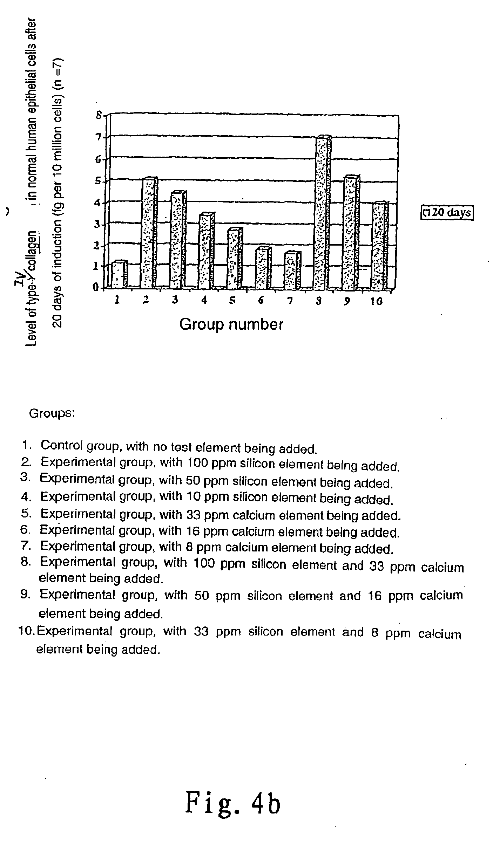

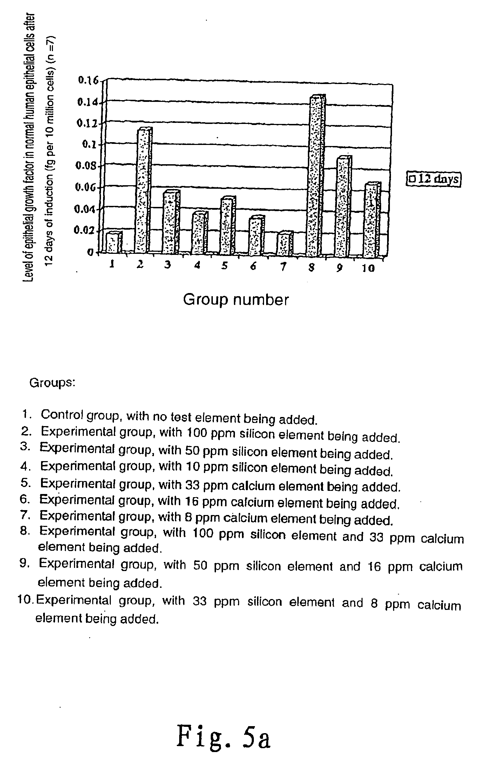

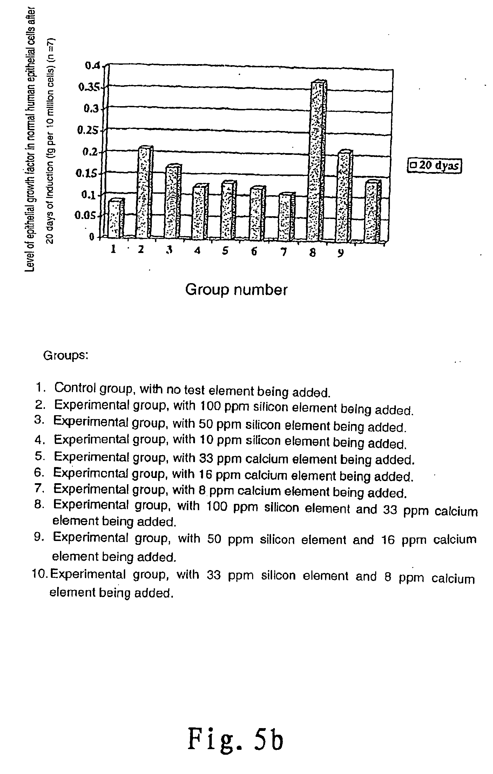

For the first time, the inventor proves and uses the inorganic element "silicon" as the main chemical component for inducing the proliferation and differentiation of human epithelial cells. The experimental data of normal human epithelial cells as set forth in the figures prove that the silicon added in the cell culture media exhibits an activity (1-6 fold) of promoting the proliferation of epithelial cells (see Fig. 3), the synthesis and secretion of type-IV collagen(see Fig. 4), and the synthesis and secretion of epithelial growth factor (see Fig. 5), which are key biological indexes of the proliferation and differentiation of epithelial cells. Hence, in the present invention, the inorganic element "silicon" is the main chemical substance for inducing the proliferation and differentiation of human epithelial cells.

For the first time, the inventor further proves and uses inorganic element "calcium" as a chemical component for inducing the proliferation and differentiation of human epithelial cells and for neutralizing the acid products generated on the wound surface of skin. According to the experimental data shown in Fig. 3, Fig. 4 and Fig. 5 (see below), it is proven that the calcium added in the cell culture media exhibits inducing activity (1-2 fold) of promoting the proliferation of epithelial cells (see Fig. 3), the synthesis and secretion of type-IV collagen(see Fig. 4), and the synthesis and secretion of epithelial growth factor (see Fig. 5), which are key biological indexes of the proliferation and differentiation of epithelial cells. Hence, in the present invention, the inorganic element "calcium" is defined as another effective chemical substance for inducing the proliferation and differentiation of human epithelial cells.

In the dressing material of the present invention, the inorganic elements silicon and calcium can be used alone or in combination. For the first time, the inventor proves that the addition of calcium in cell culture media containing silicon can increase the proliferation of epithelial cells (see Fig. 3), the synthesis and secretion of type-IV collagen(see Fig. 4), and the synthesis and secretion of epithelial growth factor (see Fig. 5) for 6-7 folds, demonstrating a significant synergistic inducing effect.

In the present invention, unless otherwise stated, silicon calcium elements can be used alone to induce the proliferation and differentiation of human epithelial cells, so as to induce the new bone formation actively and effectively.

Thus, unless otherwise stated, silicon and calcium elements can be used in combination in the present invention to form an "element combination having effective biological induction activity", wherein the atomic proportions of said elements in the combination are: silicon from 0 to 100%, and calcium from 100 to 0%. In the present invention, the "element combination of substances having effective biological induction effect" with optimal inducing activity contains silicon from 50 to 100%, and calcium from 0 to 50%, in percentage of atomic weight.

For the first time, the inventor further proves that, according to the experimental data in Fig. 3, Fig. 4 and Fig. 5, the exhibited biological induction effect is directly proportional to the concentrations of silicon and calcium, and the maximum biological induction effect is achieved at the saturation physiological concentrations of silicon (100 ppm) and calcium (33 ppm) respectively. Thus, in the present invention, the concentrations of the elements having biological induction effect in the dressing material are: for silicon, the concentration thereof is effective to increase the silicon concentration in wound surface tissue to 1 ppm to 100 ppm, and for calcium, the concentration thereof is effective to increase the calcium concentration in wound surface tissue to 1 ppm to 33 ppm.

The term "individual" in the present invention represents an animal, in particular a mammalian (such as an ape, cattle, horse, pig, sheep, rodent, goat, dog, cat, rabbit), preferably human.

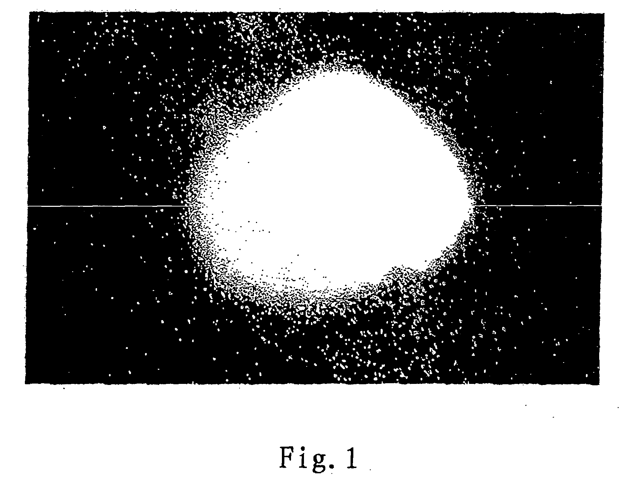

The silicon and calcium microparticles in the biological active material can be various microparticles containing one of these two elements, a combination of the microparticles of these two elements, or composite microparticles obtained by mixing these two elements and drying and milling the resultant mixture via conventional physical or chemical procedures. Fig. 1 is a scanning electron micrograph of a dressing material comprising silicon/calcium microparticles.

In the present invention, the atomic ratio of the mixed microparticles containing one of these two elements or of these two elements in the composite microparticles is not critical for achieving the object of the present invention, because all atomic ratios or weight ratios of these two elements are within the range of exhibiting effective induction effect, and the difference merely lies in the level of inducting activity. Hence, all combinations containing inorganic element silicon as biological inducing agent alone or in combination with inorganic element calcium as synergistic active substance in any atomic or weight ratio can be used as biological active agents in the wound dressing material for human skin of the present invention.

The diameters of silicon and calcium microparticles can be in a mixed-type distribution (see Fig. 1), and the diameters of silicon and calcium microparticles are generally in the range of from 100 nm to 100 µm, wherein small microparticles (less than 1000 nm) have advantages of immediately exhibiting the biological induction effect when the microparticles contact with cells or wound surface of skin, while large microparticles (greater than 1000 nm and less than 100 µm) bring about slow and long-term biological induction effect by gradually dissolving and releasing after the microparticles contact with cells or wound surface of skin. Thus, the mixture of large and small microparticles can not only bring about immediate induction effect to epithelial cells in the wound surface, but also maintain the proliferation and differentiation of the epithelial cells ensuring the sustained induction effect of dressing material on the wound surface. It should be noted that the size of microparticles in the present invention is merely associated with the time for bringing about the induction effect and the time for sustaining the induction effect, and does not directly change the biological induction activity of the silicon/calcium material as defined in the present invention. Hence, in the present invention, the silicon and calcium microparticles can be of any diameter which permits said microparticles to be soluble. Preferably the diameters of silicon and calcium microparticles are in the range of from 100 nm to 100 µm.

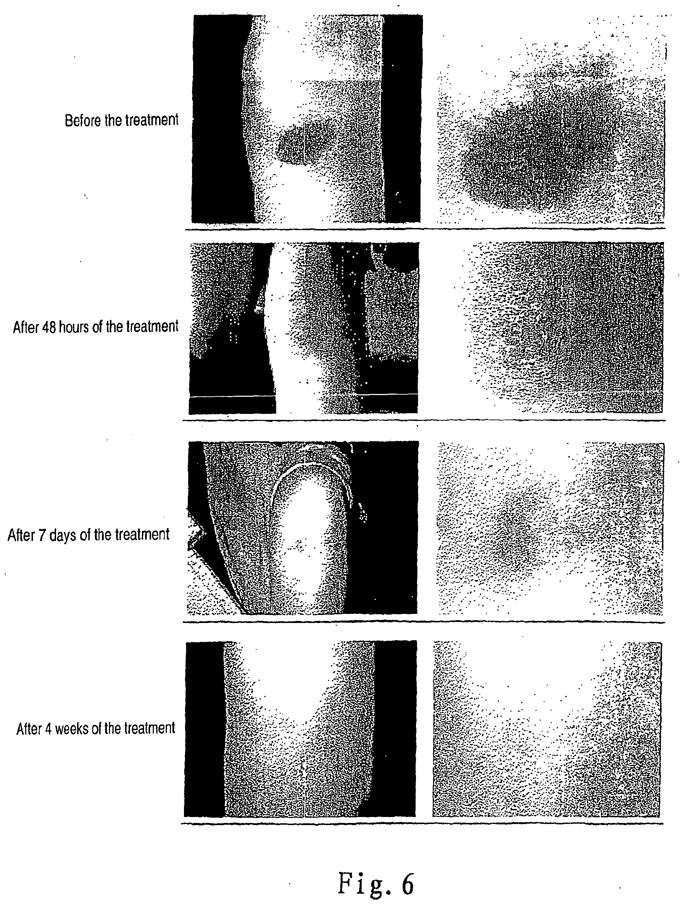

In the present invention, inorganic elements silicon and calcium are determined to be biologically active elements capable of inducing the proliferation, differentiation and repair of human skin tissue, which is a break-through in the field of biological material. In the prior art, synthesized or extracted exogenous collagens, epithelial growth factors or various adhesive proteins are considered to have epithelia induction effect, but they have some shortcomings, such as less safety, inferior stability of biological activity and high cost, and can hardly be used in biological engineering or clinically. According to the experimental data in Fig. 3, Fig. 4 and Fig. 5, as well as the clinical case of human skin wound shown in Fig. 6 of the present invention, it is firstly proven that inorganic elements silicon and calcium alone or in combination can replace biologically active protein to induce the epithelial cells and to obviously promote the rapid healing of wound surface of skin. The use of these inorganic elements as main active agents in a dressing material for wound surface of human skin has advantages of safety, stability, readily obtaining, and low cost, and can obviously increase the practical applicability of the dressing material for wounded skin.

In the prior art, small wounds of skin surface in daily life can be treated with sterilized adhesive plasters such as "Bandages". However, these adhesive bandages is used only to cover wound surface, and have no activity of anti-inflammation, inducing the growth of epithelia or promoting the rapid healing of wounded skin. In the prior art, single or composite antibiotics are also used in dressings for treatment of wounded skin, but these dressings can merely control the secondary bacterial infection at the wound surface, but cannot initiatively induce the rapid growth of epithelial cells at the wound surface to result in the rapid wound healing. The wound dressing materials containing inorganic element silicon and/or calcium in the present invention can be used in various manners, including but not being limited to the following three manners:
1) In form of microparticles powder, being directly applied to the wound surface of skin, wherein the composition of the microparticles is as defined in the present invention, i.e., the microparticles comprise various microparticles containing one of silicon and calcium elements, a combination of the microparticles of these two elements, or composite microparticles obtained by mixing these two elements and drying and milling the resultant mixture via conventional physical or chemical procedures, in combination with a pharmaceutically acceptable carrier, excipient and/or adjuvant.
2) The silicon and/or calcium inorganic elements can be formulated into ointment by mixing with conventional adjuvant such as medical Vaseline as base, which is useful for treating the wound surface of skin. The base can cover the wound surface and also act as a carrier to carry effective agents such as the biologically active inorganic elements as mentioned above and to permit the effective agents to adhere to the wound surface of skin for a long period and to achieve the optimal biological effect. The contents of the biological induction inorganic elements in the ointment are as defined in the present invention, i.e., the concentration of silicon is such that permits to increase the silicon concentration in wound surface tissue to 1 ppm to 100 ppm, and the concentration of calcium is such that permits to increase the calcium concentration in wound surface tissue to 1 ppm to 33 ppm.
3) A "patch" - type skin wound dressing can be formulated by using the silicon and/or calcium inorganic microparticles as defined in the present invention as biological induction component and a conventional gauze of adhesive plaster as substrate. The effective biological induction components of silicon and calcium inorganic elements in the patch-type skin wound dressing are as defined in the present invention above in terms of chemical species, proportions and particulate diameters. The patch-type skin wound dressing can be of any size suitable for various human skin wounds.

The dressing material of the present invention can be used in many fields, for example, it can be used as coating layer on the surface of various devices (such as metal stents for coronary artery) to be implanted into human body.

The dressing material of the present invention may further comprise no or any conventional broad-spectrum or specific antibiotic in any amount, and no or any conventional topical anesthetic in any amount.

The dressing material of the present invention may further comprise other factors which are capable of promoting the proliferation of epithelial cells, such as collagens, epithelial growth factor and/or human growth factors, various natural proteins extracted from human body, various antibiotics and bacteriostatic agents in the form of Chinese traditional medicine or Western medicine, various anti-inflammatory agents, various autologous or xenogenous skin materials, various animal skin materials or extracts thereof, various metals and organic additives, or any combination thereof.

The benefits of the present invention as compared to the prior art lie in: the use of silicon and calcium microparticles with induction effect to human epithelial cells as main effective component of "biological induction active substance" in the dressing material causes the dressing material of the present invention to be obviously superior to those conventional "Bandages" and antibiotic ointments without biological induction effect in the prior art; due to the first use of inorganic elements as biological induction components, their biological stability and safety are obviously superior to those synthesized collagens or growth factors in the art; and since the "biological induction active substance" of the present invention is basic, it can effectively neutralize the acidic exudates from the wound surface to form a local environment facilitating the growth of epithelial cells and the rapid wound healing. The inorganic elements being used as active substances in the present invention have advantages of being stable in properties and of low cost, thus being obviously superior to any dressing material containing biological proteins as active substances in the prior art. In addition, the combination of inorganic active substances with broad-spectrum antibiotic in the present invention can effectively prevent the secondary bacterial infection on the wound surface of skin, ensure the induction effect of silicon/calcium active elements on epithelial cells and the rapid healing of the skin. In sum, the dressing material of the present invention can initiatively induce the proliferation of epithelial cells and promote the rapid wound healing (the period being shortened at least by half). The rapid healing of the wound surface may prevent or reduce the formation of scar after the healing of the wounded skin. Thus, the dressing material of the present invention is suitable for the treatment of various wound surfaces of skin, for example large surface burns, chemical burns, bedsores and skin ulcers of patients suffering for example diabetes, and various incised wounds, injuries from falls, contusions, scalds in daily life, and the like.

These novel dressing materials have biological induction activity, and also have the advantages of stable property, safety, low cost and facility in application, thus can be effectively and widely used on various wound surfaces of skin, ranging from small surface wounds such as incised wounds and contusions in daily life to large surface wounds such as burns, chemical burns, bedsores and ulcers of human surface (e.g., skin ulcers of patients suffering diabetes) to promote the rapid wound healing.

The biologically active components of the skin dressing material of the present invention differ from the topical wound therapeutic formulation containing a hyaluronic acid as main active substance as disclosed in US Patent 6262020 in that, the biological active components of the present invention can both control inflammation and promote the growth of epithelia, while the hyaluronic acid can merely control the inflammatory reaction at the wound surface of skin. The product of the present invention differs from the serine protease inhibitors as disclosed in US Patent 5,190,917, US Patent 5,290,762 and US Patent 6,262,020 in that, these serine protease inhibitors merely have the anti-inflammatory effects, but have no biological induction effect as the product of the present invention. The present invention differs from the topical wound dressing comprising plasma fibronectin as disclosed in US Patent 5,604,200 in that, although the dressing disclosed in US Patent 5,604,200 could theoretically promote the adhesion of human cells, but in practical application, if the plasma fibronectin is extracted from human plasma, the source will be limited and the plasma fibronectin will be too expensive to be used in clinic, and if the fibronectin is obtained via genetic engineering process in vitro, the proteins obtained thereby will not possess the biological activity similar to that of the natural proteins in human body. Hence, the theory of using a protein with biological activity as an active ingredient in a common topical formulation is lack of practical clinical value. In the conventional concepts, all inorganic element materials are "inert", i.e., inorganic element materials do not possess the activity of stimulating the growth of organisms. The active chemical components used in the dressing material of the present invention is a group of inorganic element materials, and for the first time the present inventor discovers that some inorganic elements have biological induction activity to epithelial cells. Due to this discovery, a combination of inorganic elements can be used to replace conventional biological induction proteins to promote the induction of growth of epithelial cells. In the meantime, said combination of inorganic elements further has anti-inflammatory effect and perfect biological stability, so that it can solve the problems existing in the prior art and can be used to form surface dressing materials having functions of relieving inflammation, inhibiting bacteria, promoting the proliferation of epithelial cells and repairing wound surface for the treatment of incised wounds, contusions, burns, chemical burns etc.

In the present invention, unless otherwise stated, the term "biological induction active substance" is defined as a substance being capable of initiatively stimulating the normal proliferation and differentiation of cells to achieve specific physiological functions. The silicon and calcium inorganic elements of the present invention have the biological induction activity of initiatively stimulating normal proliferation and specific differentiation of human epithelial cells to achieve specific physiological functions (such as the synthesis and secretion of type-IV collagen and epithelial growth factor). The synthesis and secretion of type-IV collagen and epithelial growth factor plays a significant function in the rapid proliferation of epithelial cells and the rapid repair of wound surface of skin. No dressing materials for wound surface of skin in the prior art possesses such a specific biological induction function. The "biological induction active substance" of the present invention is further defined as inorganic elements silicon and calcium.

The present invention further provides a method of promoting the proliferation of epithelial cells in vitro, comprising applying an effective amount of silicon and/or calcium microparticles of the present invention to such epithelial cells. In an embodiment, said microparticles are soluble and have a diameter of for example from 100 nm to 100 µm. Said microparticles can be in any form of inorganic silicon and calcium substances, including SiO₂, NaAlSiO₂, KAlSiO₂, CaO, CaSO₄, CaPO₄, CaCl₂ etc. or any combination thereof. In one embodiment, said silicon and calcium microparticles are used in a combination, wherein silicon comprises 50-100% and calcium comprises 0-50%, in terms of relative atomic weight. The final concentration of silicon can be for example 1-100 ppm, and the final concentration of calcium can be for example 1-33 ppm.

The present invention is further described in detail with the following non-limiting examples together with the drawings.

### EXAMPLE 1

### Preparation of mixed-type biological induction silicon/calcium microparticles

The diameters of silicon and calcium microparticles of the present invention can be in a mixed-type form. In one embodiment, the diameter of silicon and calcium microparticles of the present invention is in the range of from 100 nm to 100 µm, but the present invention is not limited to this range. The starting materials are silica (SiO₂) and calcia (CaO), and the weight ratios of the starting materials are: silica 60% and calcia 40%. The process for the preparation of the mixtures comprises: mixing said silicon and calcium inorganic starting materials in a predertermined ratio, grinding in a Retsch mill for 1 to 3 days, screening with fine mesh screens to obtain various microparticles with different diameters in the range from 100 nm to 100 µm, and mixing these microparticles according to their size and proportion. The diameters of microparticles are determined by the measurement of scanning electron microscope. Fig. 1 is a scanning electron micrograph of a dressing material comprising silicon/calcium microparticles. These mixed-type biological induction silicon/calcium microparticles are autoclaved, and are used in the cytological tests and the clinical case in the following examples.

### EXAMPLE 2

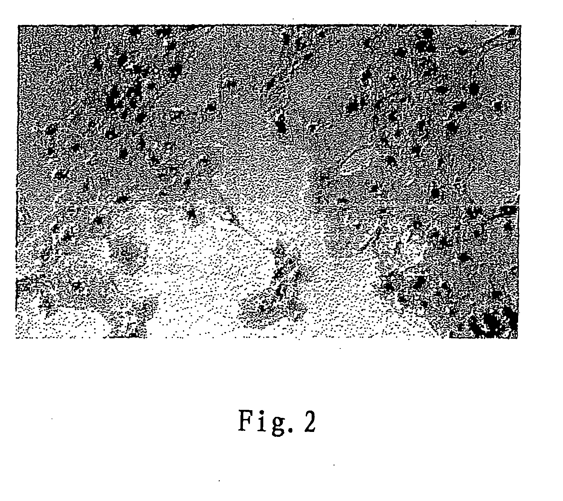

### Proliferation and migration of normal human epithelial cells under the induction of silicon and calcium microparticles (Fig. 2)

Human epithelial cells to be tested are taken from skin tissues of 20-25 aged healthy donors. The epithelial tissues are washed with sterilized physiological saline, and placed in cell culture dishes. These culture dishes were then placed in a conventional cell culture medium containing 10% fetal bovine serum and 0.2% broad-spectrum antibiotic, then the dishes are incubated in a 5% CO₂ incubator at 37°C, The medium is replaced with a fresh medium daily. On the 14^{th} day, the epithelial cells migrate from the cultured epithelial tissues to the surfaces of the culture dishes. Then the tissues are removed, and the derived epithelial cells are maintained on the culture dishes to proliferate. When the cells are passaged for three times or proliferate to a sufficient number, the cells are separated from the culture dishes by 10% collagenase to be used in subsequent tests. In the tests, the cells were seeded on conventional culture dishes of a diameter of 2 cm in a medium containing 10% fetal bovine serum and broad-spectrum antibiotic as well as silicon/calcium inorganic element microparticles prepared according to Example 1. During the culture procedure, the media are replaced with fresh media having the same compositions every four days, and then the relevant physiological activities of the cells are measured at the time points as described in these examples. In the photograph of the present example, it can be seen that epithelial cells proliferate significantly, extend and migrate on the second day of cell culturing.

### EXAMPLE 3

### Notable induction effect of silicon and calcium microparticles on the proliferation of normal human epithelial cells (Fig. 3)

The early rapid proliferation of epithelium is a pre-requisite for the rapid healing of wound surface of epithelium. In the present tests, the epithelial cells used for identifying test materials are taken from normal human skin, and the cells are cultured and grow in media containing the silicon and calcium microparticles of the present invention prepared according to the process of the Example 2. The specific combinations of chemical ingredients are pre-added in to the cell culture media used in the present tests at the concentrations as depicted in the Table of Fig. 3. One group serves as a control. Experimental groups with the test materials are compared to said control group to determine the induction effect of the test materials on the growth of cells. During the culture procedure, the media are replaced with fresh media having the same specific compositions every four days. Cell proliferation tests are conducted on the 12^{th} and 20^{th} day by counting the total number of cells grown in media containing various combinations of additives at various concentrations by a conventional flow cytometer, and the proliferation folds of the epithelial cells as shown in Fig. 3 were calculated based on the cell numbers adhered to the culture dishes during the first 24 hours. The tests prove that inorganic element silicon exhibits an obvious activity of inducing the proliferation of epithelial cells. As compared to the control group, on the 12^{th} day, 100 ppm silicon results in approximate 3-fold proliferation, 50 ppm silicon results in approximate 2.5-fold proliferation, and 10 ppm results in approximate 1.5-fold proliferation; 33 ppm calcium results in approximate 1.5-fold proliferation, and 16 ppm and 8 ppm calcium also result in obvious proliferation (see Fig. 3a). When these two inorganic elements silicon and calcium are added into the media at the various concentrations as shown in Fig. 3b, the cell proliferation are increased by 4.5-fold, 3.5-fold and 2-fold in comparison with the control group respectively. On the 20^{th} day, due to the sustained induction effect, 100 ppm silicon induces 3-fold proliferation, and the combination of 100 ppm silicon and 33 ppm calcium results in more than 5-fold proliferation. From the results of the groups with different combinations of chemical components during different culture periods, it can be seen that the induction effect of the cell proliferation are directly proportional to the concentrations of silicon and calcium within the ranges applied in the present tests. Thus, it is proven that inorganic elements silicon and calcium exhibit obvious induction effect on the proliferation of epithelial cells.

### EXAMPLE 4

### Silicon and calcium microparticles significantly induce the synthesis and secretion of type-IV collagen in normal human epithelial cells(Fig. 4)

Type-IV collagen is one the main extracellular matrix proteins in epithelium, and its synthesis and secretion is one of the important indexes of the activity of normal human epithelial cells. It plays an important role in the healing of wound surface of epithelium. The increase of synthesis and secretion of type-IV collagen facilitates the rapid healing of wound surface of epithelium. In the present example, the normal human epithelial cells used in the experimental groups and control group are cultured in accordance with the procedure and conditions of the Example 3. The only difference lies in that the synthesis and secretion of type-IV collagen of the cultured epithelial cells are quantitatively measured on the 12^{th} day and 20^{th} day during the culture. The level of type-IV collagen secreted into the cell culture media by the cells of these groups were determined by conventional immunohistochemical methods with the monoclonal antibody against type-IV collagen (commercially obtained from Sigma Inc). The measurements are expressed by fg of type-IV collagen secreted by per 10 millions cells on the 12^{th} day and 20^{th} day. The results demonstrate that the inorganic element silicon significantly induces the synthesis and secretion of type - IV collagen. As compared to the control group, on the 12^{th} day during the culture., 100 ppm silicon results in 1-fold increase, and 50 ppm and 100 ppm silicon also results in significant increases; 33 ppm calcium results in 0.5-fold increase, and 16 ppm and 8 ppm calcium also results in significant increases. When these two inorganic elements silicon and calcium are added into the culture media at the various concentrations as shown in Fig. 4, the synthesis and secretion of type-IV collagen respectively increase by 2-fold, 1.5-fold and 1-fold in comparison with the control group. On the 20^{th} day, due to the sustained induction effect of the test material, 100 ppm silicon increases the synthesis and secretion of type-IV collagen by 4-fold, and the combination of 100 ppm silicon and 33 ppm have an induction effect of more than 6 folds. From the results of different combinations of chemical components during different culture periods, it can be seen that the induction effect on the synthesis and secretion of type-IV collagen are obviously directly proportional to the concentrations of silicon and calcium within the ranges of tested concentrations and combinations. Thus, it is proven that inorganic elements silicon and calcium exhibit obvious induction effect on the synthesis and secretion of type-IV collagen in epithelial cells. The sustained and gradually increasing of the level of type-IV collagen is of great advantage to the construction of collagen matrix in the wound surface as well as the healing of wound surface.

### EXAMPLE 5

### Silicon and calcium microparticles induce significantly the synthesis and secretion of epithelial growth factor in normal human epithelial cells (Fig. 5)

Epithelial growth factor is one of the main regulatory proteins in the proliferation of epithelial cells, and its synthesis and secretion is one of the important indexes of activity of normal human epithelial cells. Epithelial growth factor plays an important role in the healing of wound surface of epithelium. The increase of the synthesis and secretion of epithelial growth factor facilitates the rapid proliferation of epithelial cells and the rapid healing of wound surface. In the present test, normal human epithelial cells used in the experimental groups and the control group are cultured in accordance with the procedure and conditions as disclosed in Example 4, the differences being that the synthesis and secretion of epithelial growth factor of the cultured epithelial cells are quantitatively measured on the 12^{th} day and 20^{th} day during the culture period. The contents of epithelial growth factor secreted by cells of these groups into the cell culture media are measured by conventional immunohistochemical methods with the monoclonal antibody against epithelial growth factor (commercially obtained from Sigma Inc), and the values are expressed as fenograms of type-IV collagen secreted by per 10 millions cells on the 12^{th} day and 20^{th} day. The test results prove that inorganic element silicon exhibits significant activity of inducing the synthesis and secretion of epithelial growth factor. As compared to the control group, on the 12^{th} day during the culture period, 100 ppm silicon results in about 4.5-fold increase, and 50 ppm and 10 ppm silicon also results in significant increases; 33 ppm calcium results in about 1.5-fold increase, and 16 ppm calcium results in obvious increases as well. When these two inorganic elements silicon and calcium are added into the media at the various concentrations as shown in Fig. 5, the synthesis and secretion of the epithelial growth factor respectively increase by 6.5-fold, 3.5-fold and 2.5-fold in comparison with the control group. On the 20^{th} day during the culture period, due to the sustained induction effect of the test material, 100 ppm silicon increases the synthesis and secretion of the epithelial growth factor by one fold, and the combination of 100 ppm silicon and 33 calcium ppm results in an induction effect of near 3-fold. From the results of the groups with different combinations of chemical components during different culture periods, it can be seen that the induction effect on the synthesis and secretion of the epithelial growth factor is directly proportional to the concentrations of silicon and calcium within the ranges of the tested concentrations and combinations. Thus, it is demonstrated that the inorganic elements silicon and calcium exhibit obvious induction effect on the synthesis and secretion of the epithelial growth factor in epithelial cells. Especially, the great increase of the synthesis and secretion of the epithelial growth factor induced by the test materials during the early culture period significantly enhances the proliferation and amplification of the epithelial cells at the early stage of the healing of wounded epithelium, and vastly promotes the healing of wound surface.

### EXAMPLE 6

### Clinical case of using a novel dressing material of silicon and calcium microparticles for promoting the rapid healing of wound surface on human skin

The present example illuminates the clinical effect of the novel dressing material of the silicon and calcium microparticles on promoting the rapid healing of wound surface on human skin. The patient is a 16-year old girl, who injured her left knee when falling near a swimming pool. The contusion had an area of about 3 x 6 square centimeter and reached the deep layer of dermis with obvious exudates on the wound surface. After being hurt, the patient was immediately treated by spraying the novel dressing material of the silicon and calcium microparticles on the wound surface, with a sterilized gauze covered thereon. After 48 hours from the treatment, the exudates from the wound surface completely disappeared, no secondary infection occurred on the wound surface, and most of the wound surface were covered with epithelial cells. After 7 days of treatment, the wound surface was completed repaired, and the skin surface was flat, except for a small amount of residual heme caused by blood oozing in the contusion. A reexamination carried out after 4 weeks of the treatment showed the wound surface was completely healed, the retained heme substantively disappeared, and there was no notable pigmentation and no scar. Fig. 6 showed photographs of wound surface at different time points after the treatment with the novel dressing material powder of the present invention, with magnified photographs shown in the right column. The present example proves that the wound repairing capability of the novel dressing material of the present invention is very significant. When the wound surface is treated with a conventional dressing material, the exudates on the wound surface will last for more than 3 days, and more than 10 days will be needed for the wound surface to be covered with epithelia. Moreover, it is difficult to control the bacterial infection with a conventional dressing material, and the infection may further delay the repair of wound surface. However, the period for the wound surface to be healed will be shortened at least by half when the dressing material of the present invention is employed. Since the delay of the wound surface healing is the main factor which causes the formation of scar after the wound surface healing, the novel dressing material of the present invention that can accelerate the wound surface healing could effectively prevent the formation of scar after the wound surface healing.

It is obvious that the present invention can be carried out not in the same way as specifically disclosed in the above contents and examples.

Since the present invention may be altered or adapted based on the above teachings, the present invention can be carried out not in the same way as the specific description. All these variations fall into the scope as defined in the pending claims.

The entire contents of all the publications (including patents, patent applications, journals, laboratory manuals, books or other documents) are referred to herein by references.

### References:

1. Chou L, Firth JD, Uitto V-J, Brunette DM. (1995) Substratum surface topography alters cell shape and regulates fibronectin, mRNA level, mRNA stability, secretion and assembly in human fibroblasts. Journal of Cell Sciences. 108:1563-1573.
2. Chou L, Firth JD, Uitto V-J, Brunette DM. (1996) Effects of titanium on transcriptional and post-transcriptional regulation of fibronectin in human fibroblasts. Journal of Biomedical Materials Research. 31:209-217.
3. Chou L, Firth JD, Uitto V-J, Brunette DM. (1998) Effects of titanium substratum and grooved surface topography on metalloproteinase-2 expression in human fibroblasts. Journal of Biomedical Materials Research. 39:437-445
4. Chou L, Marek B, Wagner WR. (1999) Effects of hydroxyapatite coating crystallinity on biosolubility, cell attachment efficiency, and proliferation in vitro. Biomaterials. 20:977-985.
5. US Patent 6,262,020, July 17, 2001
6. US Patent 5,604,200, February 18, 1997

*Annotation: CHOU L. is the English name of the present inventor.

## Claims

1. A dressing material for treating or alleviating diseases or conditions of an individual in need of promoting the proliferation or differentiation of epithelial cells, wherein said dressing material comprises a therapeutically effective amount of inorganic elements silicon and/or calcium microparticles, and an optional pharmaceutically acceptable carrier and/or excipient.

2. A dressing material according to claim 1, wherein said dressing material comprises both inorganic elements silicon and calcium microparticles.

3. A dressing material according to claim 1, wherein said silicon and/or calcium microparticles are present in the form of single element microparticles or composite elements microparticles.

4. A dressing material according to claim 1, wherein said microparticles are soluble.

5. A dressing material according to claim 1, wherein said microparticles have a diameter of from 100 nm to 100 µm.

6. A dressing material according to claim 1, wherein said inorganic element silicon is selected from any silicon-containing compound such as SiO₂, NaAlSiO₂, KAlSiO₂ etc., or any combination thereof.

7. A dressing material according to claim 1, wherein on the basis of relative stoichiometric percentage, the content of silicon is 0-100%, and the content of calcium is 0-100%.

8. A dressing material according to claim 1, wherein said inorganic element calcium is present in the form of any calcium-containing compound such as CaO, CaSO₄, CaPO₄, CaCl₂ etc., or any combination thereof.

9. A dressing material according to claim 1, wherein said dressing material further comprises antibiotics, conventional topical anesthetic drugs, or other factors being capable of promoting the proliferation of epithelial cells, such as collagens and/or epithelial growth factor, or any combination thereof,.

10. A dressing material according to claim 1, wherein said dressing material is present in the form of powder, ointment or patch.

11. A dressing material according to claim 1, wherein said dressing material is a dressing material for the wound surface of skin to be used to promote the repair or healing of the wound surface of skin.

12. A dressing material according to claim 1, wherein said diseases or conditions are selected from the group consisted of incised wounds, contusions, burns, scalds, chemical burns, bedsores, various ulcers on the surface of skin, and the like.

13. A dressing material according to claim 1, wherein said individual is an animal, in particular a mammalian (such as an ape, cattle, horse, pig, sheep, rodent, goat, dog, cat, rabbit), preferably a human.

14. Use of inorganic elements silicon and/or calcium in the manufacture of a dressing material for treating or alleviating diseases or conditions of an individual in need of promoting the proliferation or differentiation of epithelial cells.

15. Use according to claim 14, wherein said dressing material is used as a surface coating layer of various devices to be implanted in vivo.

16. Use according to claim 15, said devices to be implanted in vivo is metal stents for coronary artery.

17. Use according to claim 14, wherein said diseases or conditions are selected from the group consisted of incised wounds, contusions, burns, scalds, chemical burns, bedsores, various ulcers on the surface of skin, and the like.

18. Use according to claim 14, wherein said inorganic elements silicon and/or calcium are microparticles having a diameter of from 100 nm to 100 µm.

19. A method of promoting the proliferation of epithelial cells in vitro, wherein said method comprises applying a proliferation effective amount of silicon and/or calcium microparticles to said epithelial cells.

20. A method according to claim 19, wherein the final concentration of silicon is from 1 to 100 ppm.

21. A method according to claim 19, wherein the final concentration of calcium is from 1 to 33 ppm.

22. A method according to claim 19, wherein said microparticles have a diameter of from 100 nm to 100 µm.

23. A method according to claim 19, wherein said inorganic elements silicon and/or calcium is any silicon-containing and/or calcium-containing compound such as SiO₂, NaAlSiO₂, KAlSiO₂, CaO, CaSO₄, CaPO₄, CaCl₂ etc., or any combination thereof.
